# EUROPÄISCHE PATENTSCHRIFT

(11) **EP 0 988 554 B1**
(45) Veröffentlichungstag und Bekanntmachung des Hinweises auf die Patenterteilung: **02.10.2002**
(21) Anmeldenummer: 98936135.7
(22) Anmeldetag: 12.06.1998
(51) Int. Cl.: G01P 3/48

(54) **VERFAHREN ZUR BESTIMMUNG DER DREHZAHL EINER VERBRENNUNGSKRAFTMASCHINE**
METHOD FOR DETERMINING THE ROTATIONAL SPEED OF AN INTERNAL COMBUSTION ENGINE
PROCEDE POUR DETERMINER LA VITESSE DE ROTATION D'UN MOTEUR A COMBUSTION INTERNE

(30) Priorität: 13.06.1997 DE 19725090
(43) Veröffentlichungstag der Anmeldung: 29.03.2000
(73) Patentinhaber: ROBERT BOSCH GMBH, 70442 Stuttgart (DE)
(72) Erfinder: ANLAUF, Juergen, D-73035 Göppingen (DE); GERDES-ROEBEN, Michael, D-73061 Ebersbach (DE); SIEG, Raymond, D-73732 Esslingen (DE); FISCHER, Uwe, D-71409 Schwaikheim (DE); LEHMANN, Siegfried, D-73061 Ebersbach (DE); WIDMANN, Klaus, D-71686 Remseck (DE); NOBIS, Guenter, D-73240 Wendlingen (DE)
(86) Internationale Anmeldenummer: DE9801588
(87) Internationale Veröffentlichungsnummer: WO98058263

(56) Entgegenhaltungen:
- EP-A- 0 133 426
- EP-A- 0 408 877
- EP-A- 0 701 134
- DE-A- 3 832 518
- US-A- 5 581 178

## Beschreibung

### Stand der Technik

Die Erfindung geht aus von einem Verfahren zur Bestimmung der Drehzahl einer Verbrennungskraftmaschine, die einen Generator enthält, der von der Verbrennungskraftmaschine angetrieben wird, und mit einer signalverarbeitenden Anordnung, der im Oberbegriff des Anspruchs 1 definierten Gattung.

Ein derartiges Verfahren ist aus der EP 0 408 877 A2 bekannt. Danach ist zur Drehzahlermittlung einer Verbrennungskraftmaschine, sei es ein Otto- oder auch ein Dieselmotor, die einen von ihr angetriebenen Generator enthält, ein Verfahren vorgesehen, bei dem eine signalverarbeitende Anordnung aus dem am Generator bzw. der Last, sowie von der Verbrennungskraftmaschine erzeugten Signalen erzeugten Signal, die Drehzahl ermittelt. Dabei wird aus Signalanteilen höherer und niedrigerer Frequenz zusammen das Übersetzungsverhältnis des Antriebs zwischen Verbrennungskraftmaschine und Generator ermittelt. Dieses Übersetzungsverhältnis geht, ebenso wie die Zylinderzahl der Verbrennungskraftmaschine, in die Berechnung der Drehzahl aus dem höherfrequenten Signalanteil ein. Aus dem niederfrequenten Signalanteil wird durch Frequenz-Demodulation der Arbeitstakt und aus diesem bei bekannter Zylinderzahl die Drehzahl der Verbrennungskraftmaschine ermittelt. Dieses Ergebnis wird zur Ermittlung des genannten Übersetzungsverhältnisses verwendet.

Bei diesem bekannten Verfahren werden zur Ermittlung der Drehzahl jeweils frequenzmodulierte Signalanteile der Generatorfrequenz ausgewertet. In der Praxis hat sich herausgestellt, daß dieses Verfahren insbesondere bei Dieselmotoren sehr zuverlässig arbeitet. Die in der Praxis jedoch vermehrt eingesetzte elektronische Dieselsteuerung, d.h. das Fahrpedal wirkt nicht mehr direkt auf mechanische, sondern über elektronisch gesteuerte Mittel auf die Motorsteuerung, hat bewirkt, daß dadurch erhebliche Störsignale auf das Bordnetz gelangen. Bei der Anwendung für Ottomotoren hat sich als nachteilig herausgestellt, daß die Modulation des niederfrequenten Signalanteils, aus dem der Arbeitszyklus der Verbrennungskraftmaschine ermittelt wird, wesentlich schwächer ausgeprägt ist.

Weiterhin ist aus der EP-A 0 701 134 ein Verfahren zur Ermittlung der Drehzahl eines Verbrennungsmotors bekannt, bei dem die Drehzahl zum einem aus der von einem Generator erzeugten Bordnetzspannung und zum anderen in dem unteren Drehzahlbereich aus der vom Verbrennungsmotor erzeugten Schwingung oder dem von ihrem Auspuff erzeugten Geräusch ermittelt wird. Das Geräusch wird über ein Mikrophon abgenommen, welches in der Nähe des Auspuffs angebracht werden muß. Die Schwingung wird über einen Beschleunigungsmesser abgenommen, der am Motor angebracht ist. Die aus der Frequenz des akustischen Geräuschs oder der Motorschwingung festgestellten Werte und die daraus errechneten Drehzahlwerte dienen der Kalibrierung der aus der Bordnetzspannung gewonnenen Drehzahl. Es wird daraus eine Konstante ermittelt, welche das Verhältnis zwischen der Frequenz der Generatorspannung und der akustischen bzw. mechanischen Frequenz angibt. Diese Konstante dient mit der Auswertung der Generatorfrequenz der eigentlichen Drehzahlangabe. Es ist klar, daß bei diesem bekannten Verfahren durch die Notwendigkeit der Bereitstellung von akustischem oder mechanisch schwingendem Signal ein größerer Aufwand getrieben werden muß, welcher darüber hinaus in der Handhabung sehr umständlich und zeitaufwendig ist.

Bei dem aus der EP 0 408 877 sowie der EP 0 472 877 bekannten Verfahren wird aus der Restwelligkeit des in das Bordnetz eingespeisten Drehstromgeneratorstroms, insbesondere aus den Kommutierungspulsen, ein Signal abgeleitet, welches angenähert einem Vielfachen der Motordrehzahl proportional ist. Zur Bestimmung des fehlenden Übersetzungsverhältnisses zwischen Generatordrehzahl und Motordrehzahl werden im Falle der EP 0 408 877 die Frequenzmodulation des Generatorsignals infolge der Motortakte, im Falle der EP 0 472 877 die Zündübersprecher auf das Bordnetz ausgewertet. Beim Verfahren gemäß der EP 0 701 134 wird die Bestimmung des Übersetzungsverhältnis insofern verallgemeinert, als die Generatorfrequenz bei mindestens einem Wert mittels eines unabhängigen Sensorsystems mit der wahren Drehzahl der Verbrennungskraftmaschine verglichen wird und auf diese Weise eine Kalibrierung erfolgt, wobei als unabhängiges Sensorsystem ein akustischer Sensor verwendet wird.

Allen vorstehend genannten bekannten Verfahren ist gemeinsam, daß die Drehzahl im fortgesetzten Meßbetrieb überwiegend aus einer einzigen Signalquelle gewonnen wird und die zweite Signalquelle nur zur gelegentlichen Kalibrierung herangezogen wird. Es ist aber zu berücksichtigen, daß den für die Drehzahlmessung nutzbaren Signalen aller Signalquellen auf ihren Übertragungspfaden unterschiedliche Störsignale überlagert werden. Im Falle des Bordnetzes werden diese hauptsächlich erzeugt durch induktive Verbraucher wie Generatorregler, Lüfterregler, Zündspule und elektronische Systeme wie die vermehrt eingesetzte elektronische Dieselsteuerung. Auch die akustische Signale werden gestört, z.B. durch die Überlagerung unterschiedlicher Übertragungspfade, durch Oberwellen oder andere Geräuschquellen wie Lüfter, andere Fahrzeuge, etc. Des weiteren ist zu berücksichtigen, daß sich das Nutz-/Störverhältnis drehzahlabhängig und von Fahrzeugtyp zu Fahrzeugtyp beträchtlich ändern kann. Der Aufwand zur Filterung der gestörten Signale ist erheblich und teilweise wirkungslos, wenn die Störer auf einer Frequenz mit dem Nutzsignal liegen. Aus diesen Gründen ermitteln die vorstehend genannten bekannten Verfahren zur Drehzahlmessung gelegentlich ein falsches Übersetzungsverhältnis, wodurch alle weiteren Drehzahlangaben falsch werden, oder sie reagieren zwischendurch auf Störimpulse und kehren dann wieder zur richtigen Anzeige zurück.

### Vorteile der Erfindung

Das erfindungsgemäße Verfahren zur Bestimmung der Drehzahl einer Verbrennungskraftmaschine mit den kennzeichnenden Merkmalen des Anspruchs 1 hat demgegenüber den Vorteil, eine einfache und zuverlässige Lösung zur Verfügung zu stellen, durch die eine erhebliche Verbesserung des Nutz-Störabstandes erreicht wird. Dadurch wird eine sehr zuverlässige Drehzahlbestimmung zur Verfügung gestellt, welche unter allen Umständen und bei allen unterschiedlichen Fahrzeugtypen zuverlässig ist. Weiterhin vorteilhaft ist, daß das Übersetzungsverhältnis zwischen der Verbrennungskraftmaschine und dem von ihr angetriebenen Generator sich beim erfindungsgemäßen Verfahrens quasi als Abfallprodukt ergibt, wohingegen es ansonsten bekannt sein muss.

Gemäß dem Verfahren nach der Erfindung werden dazu prinzipiell die Signale der verschiedenen Quellen in der signalverarbeitenden Anordnung systematisch gleichzeitig und im wesentlichen gemeinsam ausgewertet und bereits während des Auswertevorgangs durch geeignete Verfahren miteinander verknüpft.

Als auszuwertende Signale werden zum einen solche verwendet, die in irgendeiner Form Drehzahlinformationen enthalten, und zum anderen solche, die als Störsignale sowohl für sich allein meßbar, als auch der Nutzinformation überlagert sind.

Durch die in den abhängigen Ansprüchen niedergelegten Maßnahmen sind vorteilhafte Weiterbildungen und Verbesserungen des im Anspruch 1 angegebenen Verfahrens möglich.

Nach einer besonders zweckmäßigen Weiterbildung der Erfindung werden als geeignete Verfahren zur Verknüpfung allein oder in Kombination die Kreuzkorrelation, die adaptive Störunterdrückung bzw. die adaptive Rauschaustastung (Adaptive Interference Canceling), und/oder neuronale Netze verwendet.

In vorteilhafter Ausgestaltung des erfindungsgemäßen Verfahrens ist vor der gemeinsamen, gleichzeitigen Auswertung der aus verschiedenen Quellen stammenden Signale eine getrennte Aufbereitung der einzelnen Signale vorgesehen. In zweckmäßiger Ausgestaltung kann die getrennte Aufbereitung ein Filtern, eine Autokorrelation, eine Amplitudenbegrenzung oder ähnliches der einzelnen Signale sein.

Dabei werden in besonders vorteilhafter und zweckmäßiger Weiterbildung der Erfindung als Signale, die in irgendeiner Form Drehzahlinformationen enthalten, insbesondere folgende verwendet:
das Luftschall erzeugende Fahrzeuggeräusch, die Körperschall erzeugenden Fahrzeug- und/oder Motorvibrationen, die Bordnetzspannung, insbesondere Quellen und Senken, welche der Bordnetzspannung im Takt der Drehzahl oder eines Vielfachen der Drehzahl Impulse einprägen, Druckschwankungen im Ansaug- und Auspufftrakt, Öldruckschwankungen.

Entsprechend einer vorteilhaften Weiterbildung des erfindungsgemäßen Verfahrens ist vorgesehen, daß als Signale, die in irgendeiner Form Drehzahlinformationen enthalten und aus der Bordnetzspannung, insbesondere den Quellen und Senken, welche der Bordnetzspannung im Takt der Drehzahl oder eines Vielfachen der Drehzahl Impulse einprägen, solche ausgewertet werden, welche von elektrischen Einspritzventilen, von mit dem Generator und der Zündanlage verbundenen Strömen, Spannungen und elektromagnetischen Feldern herrühren. In zweckmäßiger Weiterbildung dieser Ausführungsform ist vorgesehen, daß als auszuwertende Signale, welche als Störsignale sowohl für sich allein meßbar, als auch der Nutzinformation überlagert sind, solche verwendet werden, die als Regler- oder als Lüftersignale oder als Umgebungsgeräusche auftreten und die als Störsignale identifiziert sind, und somit aus dem Gesamtsignal entfernt werden können.

Gemäß einer weiteren vorteilhaften Ausgestaltung des erfindungsgemäßen Verfahrens werden die für die Auswertung erforderlichen Rechenoperationen mit Hilfe eines Prozessors, insbesondere eines Mikroprozessors durchgeführt, der in der signalverarbeitenden Anordnung vorgesehen ist.

Entsprechend einer weiteren besonders zweckmäßigen Weiterbildung des erfindungsgemäßen Verfahrens erfolgt bei prinzipbedingt längeren Meßzeiten, insbesondere bei einer Mittelwertbildung über mehrere Meßwerte, die Mittelung bei fester Drehzahl über viele Meßwerte, während bei Drehzahländerungen dagegen die Mittelwertbildung nur über wenige Meßwerte durchgeführt wird.

### Beschreibung des Ausführungsbeispiels

Entsprechend dem prinzipiellen Kern werden beim Verfahren nach der Erfindung die Signale der verschiedenen Quellen in der signalverarbeitenden Anordnung systematisch gleichzeitig und im wesentlichen gemeinsam ausgewertet. Bereits während des Auswertevorgangs werden die Signale durch geeignete Verfahren miteinander verknüpft. Dadurch wird eine entscheidende Verbesserung des Nutz-Störabstandes erreicht und die Basis für eine vom Fahrzeugtyp unabhängige Bestimmung der Drehzahl ermöglicht.

Als geeignete Verfahren zur Verknüpfung können allein oder in Kombination die Kreuzkorrelation, die adaptive Störunterdrückung bzw. die adaptive Rauschaustastung (Adaptive Interference Canceling), und/oder neuronale Netze verwendet werden. Das Adaptive Interference Canceling ist beispielsweise ausführlich dargelegt in Kapitel 12 des Buches "Adaptive Signal Processing" von Bernard Widrow und Samuel D. Stearns, Prentice-Hall, Inc., Seite 303 bis 361. Kreuzkorrelation und neuronale Netze sind bei der Be- und Verarbeitung von Signalen allgemein bereits in relativ breiter Anwendung bekannt.

In vorteilhafter Ausgestaltung kann vor der gemeinsamen, gleichzeitigen Auswertung der aus verschiedenen Quellen stammenden Signale eine getrennte Aufbereitung der einzelnen Signale vorgesehen sein. Die getrennte Aufbereitung kann durch Filtern, Autokorrelation, Amplitudenbegrenzung oder ähnliches der einzelnen Signale durchgeführt werden.

Bei dem Verfahren nach der Erfindung werden in vorteilhafter Ausgestaltung als auszuwertende Signale zum ersten solche verwendet, die in irgendeiner Form Drehzahlinformationen enthalten, und zum zweiten solche, die als Störsignale sowohl für sich allein meßbar, als auch der Nutzinformation überlagert sind.

Dabei werden als Signale, die in irgendeiner Form Drehzahlinformationen enthalten, insbesondere folgende verwendet: das Luftschall erzeugende Fahrzeuggeräusch, die Körperschall erzeugenden Fahrzeug- und/oder Motorvibrationen, weiterhin die Bordnetzspannung, insbesondere deren Quellen und Senken, welche der Bordnetzspannung im Takt der Drehzahl oder eines Vielfachen der Drehzahl Impulse einprägen. Auch Druckschwankungen im Ansaugund Auspufftrakt sowie Öldruckschwankungen kommen hier in Frage.

Als Signale, die in irgendeiner Form Drehzahlinformationen aus der Bordnetzspannung enthalten, insbesondere deren Quellen und Senken, welche der Bordnetzspannung im Takt der Drehzahl oder eines Vielfaches der Drehzahl Impulse einprägen, werden z.B. solche ausgewertet, welche von elektrischen Einspritzventilen, von mit dem Generator und der Zündanlage verbundenen Strömen, Spannungen und elektromagnetischen Feldern sowie von elektronischen Systemen wie beispielsweise der elektronischen Dieselsteuerung EDC herrühren. Zweckmäßiger Weise werden als auszuwertende Signale, welche als Störsignale sowohl für sich allein meßbar, als auch der Nutzinformation überlagert sind, solche verwendet, die als Regleroder als Lüftersignale oder als Umgebungsgeräusche auftreten und die als Störsignale identifiziert sind. Somit können sie dann aus dem Gesamtsignal entfernt werden.

Die für die Auswertung erforderlichen Rechenoperationen werden zweckmäßiger Weise mit Hilfe eines Prozessors, insbesondere eines Mikroprozessors durchgeführt, der in der signalverarbeitenden Anordnung vorgesehen ist.

Bei prinzipbedingt längeren Meßzeiten, insbesondere bei einer Mittelwertbildung über mehrere Meßwerte, erfolgt die Mittelung bei fester Drehzahl über viele Meßwerte. Dadurch wird die Meßsicherheit erhöht. Bei Drehzahländerungen dagegen wird die Mittelwertbildung nur über wenige Meßwerte durchgeführt. Dadurch wird zwar die Meßdauer verkürzt, allerdings fällt auch die Meßsicherheit geringer aus.

Das erfindungsgemäße Verfahren bietet bei wesentlich verbessertem Nutz-Störabstand die genaue und rasche Bestimmung der Drehzahl von Verbrennungskraftmaschinen. Dies bei kontinuierlicher Bestimmung des Übertragungsverhältnisses und unabhängig vom jeweiligen Fahrzeugtyp. Damit ist eine universelle Einsatzmöglichkeit geschaffen, die keinen oder höchstens geringfügigen Adaptionsaufwand am jeweiligen Fahrzeug erforderlch macht.

## Patentansprüche

1. Verfahren zur Bestimmung der Drehzahl einer Verbrennungskraftmaschine, die einen Generator enthält, der von der Verbrennungskraftmaschine angetrieben wird, und wobei mit einer signalverarbeitenden Anordnung aus vom Generator und/oder an einer Last, sowie von der Verbrennungskraftmaschine erzeugten Signalen, bei bekannter Zylinderzahl der Verbrennungskraftmaschine, die Drehzahl der Verbrennungskraftmaschine durch Auswertung bestimmter Signale ermittelt wird, wobei
die Signale der verschiedenen Quellen in der signalverarbeitenden Anordnung systematisch gleichzeitig und im wesentlichen gemeinsam ausgewertet und bereits während des Auswertevorgangs durch geeignete Verfahren miteinander verknüpft werden, **dadurch gekennzeichnet, daß** als auszuwertende Signale verschiedener Quellen zum einen solche verwendet werden, die in irgendeiner Form Drehzahlinformationen enthalten, und zum anderen solche, die als Störsignale sowohl für sich allein meßbar, als auch der Nutzinformation überlagert sind.

2. Verfahren nach Anspruch 1, **dadurch gekennzeichnet, daß** als geeignete Verfahren zur Verknüpfung allein oder in Kombination die Kreuzkorrelation, die adaptive Störunterdrückung bzw. die adaptive Rauschaustastung (Adaptive Interference Canceling), und/oder neuronale Netze verwendet werden.

3. Verfahren nach Anspruch 1 oder 2, **dadurch gekennzeichnet, daß** vor der gemeinsamen, gleichzeitigen Auswertung der aus verschiedenen Quellen stammenden Signale eine getrennte Aufbereitung der einzelnen Signale vorgesehen ist.

4. Verfahren nach Anspruch 3, **dadurch gekennzeichnet, daß** die getrennte Aufbereitung ein Filtern, eine Autokorrelation, eine Amplitudenbegrenzung oder ähnliches der einzelnen Signale ist.

5. Verfahren nach Anspruch 1 oder einem der Ansprüche 2 bis 4, **dadurch gekennzeichnet, daß** als Signale, die in irgendeiner Form Drehzahlinformationen enthalten, insbesondere folgende verwendet werden: das Luftschall erzeugende Fahrzeuggeräusch, die Körperschall erzeugenden Fahrzeug- und/oder Motorvibrationen, die Bordnetzspannung, insbesondere Quellen und Senken, welche der Bordnetzspannung im Takt der Drehzahl oder eines Vielfachen der Drehzahl Impulse einprägen, Druckschwankungen im Ansaug- und Auspufftrakt, Öldruckschwankungen.

6. Verfahren nach Anspruch 5, **dadurch gekennzeichnet, daß** als Signale, die in irgendeiner Form Drehzahlinformationen enthalten und aus der Bordnetzspannung, insbesondere den Quellen und Senken, welche der Bordnetzspannung im Takt der Drehzahl oder eines Vielfaches der Drehzahl Impulse einprägen, solche ausgewertet werden, welche von elektrischen Einspritzventilen, von mit dem Generator und der Zündanlage verbundenen Strömen, Spannungen und elektromagnetischen Feldern herrühren.

7. Verfahren nach Anspruch 1, 5 oder 6, **dadurch gekennzeichnet, daß** als auszuwertende Signale, welche als Störsignale sowohl für sich allein meßbar, als auch der Nutzinformation überlagert sind, solche verwendet werden, die als Regleroder als Lüftersignale oder als Umgebungsgeräusche auftreten und die als Störsignale identifiziert sind, und somit aus dem Gesamtsignal entfernt werden können.

8. Verfahren nach einem der vorigen Ansprüche, **dadurch gekennzeichnet, daß** die für die Auswertung erforderlichen Rechenoperationen mit Hilfe eines Prozessors, insbesondere eines Mikroprozessors durchgeführt werden, der in der signalverarbeitenden Anordnung vorgesehen ist.

9. Verfahren nach einem der vorigen Ansprüche, **dadurch gekennzeichnet, daß** bei prinzipbedingt längeren Meßzeiten, insbesondere bei einer Mittelwertbildung über mehrere Meßwerte, die Mittelung bei fester Drehzahl über viele Meßwerte erfolgt, während bei Drehzahländerungen dagegen die Mittelwertbildung nur über wenige Meßwerte durchgeführt wird.

## Claims

1. Method for determining the rotational speed of an internal combustion engine which contains a generator which is driven by the internal combustion engine, the rotational speed of the internal combustion engine being determined by evaluating specific signals with a signal-processing arrangement, from signals generated by the generator and/or at a load, and by the internal combustion engine, given a known number of cylinders of the internal combustion engine, and the signals of the various sources in the signal-processing arrangement being systematically evaluated simultaneously and essentially jointly and already being linked to one another during the evaluation process according to suitable methods, **characterized in that**, on the one hand, signals which contain rotational speed information in some form and, on the other hand, signals which can both be measured independently as interference signals and are superimposed on the useful information are used as the signals, to be evaluated, from various sources.

2. Method according to Claim 1, **characterized in that** cross-correlation, adaptive interference cancelling and/or neural networks are used alone or in combination as suitable methods for linking.

3. Method according to Claim 1 or 2, **characterized in that** separate conditioning of the individual signals is provided before the joint, simultaneous evaluation of the signals originating from various sources.

4. Method according to Claim 3, **characterized in that** the separate conditioning comprises filtering, auto-correlation and amplitude limitation, or the like, of the individual signals.

5. Method according to Claim 1 or one of Claims 2 to 4, **characterized in that** in particular the following signals are used as signals which contain rotational speed information in some form: the vehicle noise generating airborne sound, the vehicle vibrations and/or engine vibrations generating the solid-borne sound, the voltage of the vehicle's electrical system, in particular sources and sinks which impress pulses on the voltage of the vehicle's electrical system at the clock rate at the rotational speed or a multiple of the rotational speed, pressure fluctuations in the intake and exhaust tracts, oil pressure fluctuations.

6. Method according to Claim 5, **characterized in that** pulses which originate from electrical injection valves and from currents, voltages and electromagnetic fields which are connected to the generator and to the ignition system are evaluated as signals which contain rotational speed information in some form and from the voltage of the vehicle's electrical system, in particular the sources and sinks, which impress pulses on the voltage of the vehicle's electrical system with the clock at the rotational speed or a multiple of the rotational speed.

7. Method according to Claim 1, 5 or 6, **characterized in that** signals which occur as regulator signals or as fan signals or as ambient noise and which are identified as interference signals, and can thus be removed from the composite signal, are used as signals to be evaluated which can be measured as interference signals both independently and - superimposed on the useful information.

8. Method according to one of the preceding claims, **characterized in that** the calculation operations which are necessary for the evaluation are carried out using a processor, in particular a microprocessor, which is provided in the signal-processing arrangement.

9. Method according to one of the preceding claims, **characterized in that** in the case of measuring times which are longer due to the principle, in particular in the case of an average value formation over a plurality of measured values, the averaging takes place over a large number of measured values at a fixed rotational speed, while, on the other hand, when there are changes in rotational speed the average value formation is carried out only over a few measured values.

## Revendications

1. Procédé pour déterminer le régime d'un moteur à combustion interne comportant un générateur entraîné par le moteur à combustion interne, et un montage de traitement des signaux à partir du générateur et/ou de la charge ainsi que des signaux générés par le moteur à combustion interne, dont le nombre de cylindres est connu, pour déterminer le régime (vitesse de rotation) du moteur à combustion interne par l'exploitation de certains signaux,
selon lequel on exploite essentiellement en commun et systématiquement en même temps les signaux des différentes sources dans le montage de traitement de signaux, en les combinant déjà pendant l'opération d'exploitation par des procédés appropriés,
**caractérisé en ce que**
on utilise d'une part des signaux exploités de différentes sources qui contiennent sous une forme quelconque des informations de vitesse de rotation et d'autre part des signaux qui sont des signaux parasites mesurables à la fois séparément et en combinaison avec l'information utile.

2. Procédé selon la revendication 1,
**caractérisé en ce que**
comme procédé approprié de combinaison, on utilise ensemble la corrélation croisée, la surpression adaptative des parasites ou l'élimination du bruit (suppression adaptative des parasites) et/ou des réseaux neuronaux.

3. Procédé selon la revendication 1 ou 2,
**caractérisé en ce que**
avant une exploitation commune simultanée des signaux venant de différentes sources on effectue une préparation séparée des différents signaux.

4. Procédé selon la revendication 3,
**caractérisé en ce que**
la préparation séparée consiste à filtrer, à effectuer une auto-corélation, une limitation d'amplitude ou une opération analogue sur les différents signaux.

5. Procédé selon la revendication 1 ou l'une des revendications 2 à 4,
**caractérisé en ce que**
les signaux qui contiennent sous une forme quelconque des informations relatives à la vitesse de rotation sont notamment les suivantes :
le bruit du véhicule créé sous forme de son, les vibrations du véhicule et/ou du moteur créant un bruit de structure, la tension du réseau embarqué notamment les sources et les puits qui induisent dans la tension du réseau embarqué des impulsions à la cadence de la vitesse de rotation ou à un multiple de celle-ci, des variations de pression dans la tubulure d'aspiration et la tubulure d'échappement, des variations de pression d'huile.

6. Procédé selon la revendication 5,
**caractérisé en ce que**
les signaux qui sous une forme quelconque contiennent des informations de vitesse de rotation et de la tension du réseau embarqué, notamment les utilisateurs et les sources qui impliquent des impulsions au réseau de tension embarqué à la cadence de la vitesse de rotation ou un multiple de la vitesse de rotation sont les signaux venant des injecteurs électriques, des intensités, des tensions et des champs électromagnétiques liés au générateur et à l'installation d'allumage.

7. Procédé selon les revendications 1, 5 ou 6,
**caractérisé en ce que**
les signaux à exploiter, qui se mesurent comme signaux parasites à la fois seuls et en combinaison avec l'information utile sont les signaux de régulateur ou de bruits environnants identifiés comme signaux parasites qui peuvent ainsi être dégagés du signal global.

8. Procédé selon l'une des revendications précédentes,
**caractérisé en ce que**
les opérations de calcul nécessaires à l'exploitation sont effectuées à l'aide d'un processeur notamment d'un micro-processeur du montage de traitement des signaux.

9. Procédé selon l'une quelconque des revendications précédentes,
**caractérisé en ce que**
pour des temps de mesure prolongés, liés au principe, notamment pour former une valeur moyenne sur plusieurs valeurs de mesure, on fait la moyenne pour une vitesse de rotation fixe sur plusieurs valeurs de mesure et pour une variation de la vitesse de rotation on forme la valeur moyenne seulement sur quelques valeurs de mesure.
